(19) Europäisches Patentamt
European Patent Office
Office européen des brevets

(11) EP 1 715 891 B2

(12) **NEW EUROPEAN PATENT SPECIFICATION**
After opposition procedure

(45) Date of publication and mention of the opposition decision:
**13.08.2014 Bulletin 2014/33**

(45) Mention of the grant of the patent:
**21.04.2010 Bulletin 2010/16**

(21) Application number: **05702390.5**

(22) Date of filing: **31.01.2005**

(51) Int Cl.:
***A61K 39/395*** *(2006.01)* ***A61P 19/00*** *(2006.01)*
*C07K 16/24* *(2006.01)*

(86) International application number:
**PCT/IB2005/000240**

(87) International publication number:
**WO 2005/080429 (01.09.2005 Gazette 2005/35)**

(54) **METHODS OF TREATING OSTEOARTHRITIS WITH ANTI-IL-6 ANTIBODIES**

VERFAHREN ZUR BEHANDLUNG VON OSTEOARTHRITIS MIT ANTI-IL-6 ANTIKÖRPERN

METHODES DE TRAITEMENT DE L'OSTEOARTHROSE AVEC DES ANTICORPS ANTI-INTERLEUKINE-6

(84) Designated Contracting States:
**AT BE BG CH CY CZ DE DK EE ES FI FR GB GR HU IE IS IT LI LT LU MC NL PL PT RO SE SI SK TR**

(30) Priority: **11.02.2004 US 543814 P**

(43) Date of publication of application:
**02.11.2006 Bulletin 2006/44**

(73) Proprietor: **Warner-Lambert Company LLC**
**New York, NY 10017 (US)**

(72) Inventors:
- **BOVE, Susan, Elizabeth Pfizer Global Research & Road Ann Arbor, MI 48105 (US)**
- **KILGORE, Kenneth, Stanley Pfizer Global Research**
**Plymouth Road Ann Arbor, MI 48105 (US)**

(74) Representative: **Pfizer**
**European Patent Department**
**23-25 avenue du Docteur Lannelongue**
**75668 Paris Cedex 14 (FR)**

(56) References cited:
**EP-A- 0 331 640** **WO-A-2004/039826**
**US-A1- 2003 202 977**

- **GOLDRING M B: "THE ROLE OF CYTOKINES AS INFLAMMATORY MEDIATORS IN OSTEOARTHRITIS: LESSONS FROM ANIMAL MODELS" CONNECTIVE TISSUE RESEARCH, GORDON AND BREACH SCIENCE PUBLISHERS, US, vol. 40, no. 1, 1999, pages 1-11, XP008050662 ISSN: 0300-8207**
- **MASSICOTTE F ET AL: "Can altered production of interleukin-1[beta], interleukin-6, transforming growth factor-[beta] and prostaglandin E2 by isolated human subchondral osteoblasts identity two subgroups of osteoarthritic patients" OSTEOARTHRITIS AND CARTILAGE 2002 UNITED KINGDOM, vol. 10, no. 6, 2002, pages 491-500, XP002340116 ISSN: 1063-4584**
- **BOVE S E ET AL: "Weight bearing as a measure of disease progression and efficacy of anti-inflammatory compounds in a model of monosodium iodoacetate-induced osteoarthritis" OSTEOARTHRITIS AND CARTILAGE 2003 UNITED KINGDOM, vol. 11, no. 11, 2003, pages 821-830, XP002340117 ISSN: 1063-4584 cited in the application**
- **"ATLIZUMAB ANTI-IL-6 RECEPTOR ANTIBODY - CHUGAI, ANTI-INTERLEUKIN-6 RECEPTOR ANTIBODY - CHUGAI, MRA - CHUGAI" BIODRUGS, AUCKLAND, NZ, vol. 17, no. 5, 2003, pages 369-372, XP008050680 ISSN: 1173-8804**
- **VAN ZAANEN H C T ET AL: "Chimaeric anti-interleukin 6 monoclonal antibodies in the treatment of advanced multiple myeloma: a phase I dose-escalating study" BRITISH JOURNAL OF HAEMATOLOGY, OXFORD, GB, vol. 102, 1998, pages 783-790, XP002986057 ISSN: 0007-1048 cited in the application**
- **WILDER-SMITH ET AL: 'Treatment of severe pain from osteoarthritis with slow-release tramadol or dihydrocodeine in combination with NSAID's: a randomised study comparing analgesia, antinociception and gastrointestinal effects' PAIN vol. 91, 2001, ELSEVIER SCIENCE B.V., pages 23 - 31**

EP 1 715 891 B2

## Description

### BACKGROUND OF THE INVENTION

Osteoarthritis is a disease that affects millions of people. Osteoarthirtis patients suffer from symptoms such as joint pain and joint stiffness leading to joint deformities and diminishment or loss of joint function. Aspirin and conventional nonsteroidal anti-inflammatory drugs (NSAIDs) such as ibuprofen, diclofenac, and naproxen, are typical agents used to treat osteoarthritis sufferers. EP0331640A2 describes the use of interleukin 6 in the treatment of bone conditions such as osteoporosis and osteoarthritis. There is a need in the art for additional methods of treating osteoarthritis with therapeutic agents.

### SUMMARY OF THE INVENTION

In one aspect, the present invention relates to methods of treating osteoarthritis comprising: administering, to a subject suffering from a osteoarthritis, a pharmaceutical composition comprising a pharmaceutically acceptable carrier and a therapeutically effective amount of an anti-IL-6 antibody. In certain embodiments the pharmaceutical composition is administered interarticularly or intravenously. In certain embodiments, the IL-6 antibody is a monoclonal antibody. In certain embodiments, the IL-6 antibody is CNTO 328. In certain embodiments, the present invention relates to further administering one or more agents selected from the group consisting of: 6-(5-carboxy-5-methyl-hexyloxy)-2,2-dimethylhexanoic acid calcium salt, non-steroidal anti-inflammatory agents, piroxicam, diclofenac, naproxen, flurbiprofen, fenoprofen, ketoprofen, ibuprofen, mefenamic acid, indomethacin, sulindac, apazone, phenylbutazone, aspirin, celecoxib, parecoxib, valdecoxib, etoricoxib, corticosteroids, hyalgan, and synvisc. In certain embodiments, osteoarthitic pain may be treated with an anti-IL-6 antibody. In certain embodiments, the present invention relates to methods of treating osteoarthritis comprising: administering, to a subject suffering from a osteoarthritis, a pharmaceutical composition comprising a pharmaceutically acceptable carrier and a therapeutically effective amount of an anti-IL-6 antibody.
In another aspect, the present invention relates to the use of an anti-IL-6 antibody, in the manufacture of a medicament for the treatment of osteoarthritis in subjects.

### DEFINITIONS

In a clinical setting, a physician may assess whether a patient is suffering from osteoarthritis by standard clinical indices, including radiological methods (e.g., x-rays of affected joints), and determination of The Western Ontario and McMaster Universities Osteoarthritis Index ("WOMAC") (see e.g., Creamer et al. (1999) J. Rheumatol. 26: 1785-1792).
The term "antibody" refers to a monomeric (e.g., single chain antibodies) or multimeric polypeptide comprising a framework region from an immunoglobulin gene or fragments thereof that specifically binds and recognizes an antigen. The recognized immunoglobulin genes include the kappa, lambda, alpha, gamma, delta, epsilon, and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively. The term "antibody" also includes antigen-binding polypeptides such as Fab, Fab’, $F(ab')_2$, Fd, Fv, dAb, and complementarity determining region (CDR) fragments, single-chain antibodies (scFv), chimeric antibodies, and diabodies. The term antibody includes polyclonal antibodies and monoclonal antibodies unless otherwise indicated.
An exemplary immunoglobulin (antibody) structural unit comprises a tetramer. Each tetramer is composed of two identical pairs of polypeptide chains, each pair having one "light" (about 25 kDa) and one "heavy" chain (about 50-70 kDa). The amino terminus of each chain defines a variable region of about 100 to 110 or more amino acids primarily responsible for antigen recognition. The terms variable light chain ($V_L$) and variable heavy chain ($V_H$) refer to these light and heavy chains respectively.
As used herein, a Fd fragment means an antibody fragment that consists of the $V_H$ and $C_H1$ domains; an Fv fragment consists of the $V_L$ and $V_H$ domains of a single arm of an antibody; and a dAb fragment (Ward et al., Nature 341:544-546 (1989)) consists of a $V_H$ domain.
In some embodiments, the antibody is a single-chain antibody (scFv) in which a $V_L$ and $V_H$ domains are paired to form a monovalent molecule via a synthetic linker that enables them to be made as a single protein chain. (Bird et al., Science 242:423-426 (1988) and Huston et al., Proc. Natl. Acad. Sci. USA 85:5879-5883 (1988).) In some embodiments, the antibodies are diabodies, i.e., are bivalent antibodies in which $V_H$ and $V_L$ domains are expressed on a single polypeptide chain, but using a linker that is too short to allow for pairing between the two domains on the same chain, thereby forcing the domains to pair with complementary domains of another chain and creating two antigen binding sites. (See e.g., Holliger P. et al., Proc. Natl. Acad. Sci. USA 90: 6444-6448 (1993), and Poljak R. J. et al., Structure 2:1121-1123 (1994)).

An "anti-IL-6" antibody is an antibody that specifically binds an IL-6 polypeptide. Examples of IL-6 polypeptides include, but are not limited to, a mouse IL-6 polypeptide (e.g., SEQ ID NO: 2), a rat IL-6 polypeptide (e.g., SEQ ID NO: 4), and a human IL-6 polypeptide (e.g., SEQ ID NO: 6). An example of an "anti-IL-6 antibody" is CNTO 328 (cCLB8), a human-mouse chimeric monoclonal antibody to IL-6 (see e.g., van Zaanen, et al. (1998) Br. J. Haematol. 102: 783-790).

An "anti-IL-6-receptor antibody" is an antibody that specifically binds the extracellular domain of an IL-6 receptor polypeptide. An example of an "anti-IL-6 receptor antibody" is MRA (tocilizumab). Examples of IL-6R extracellular domain polypeptides include, but are not limited to, a mouse IL-6R polypeptide (e.g., SEQ ID NO: 8), a rat IL-6R polypeptide (e.g., SEQ ID NO: 10), and a human IL-6R polypeptide (e.g., SEQ ID NO: 12).

The term " immunoassay" is an assay that uses an antibody to specifically bind an antigen. The immunoassay is characterized by the use of specific binding properties of a particular antibody to isolate, target, and/or quantify the antigen.

The phrase "specifically (or selectively) binds" to an antibody or "specifically (or selectively) immunoreactive with," when referring to a protein or peptide antigen, refers to a binding reaction that is determinative of the presence of a specified protein. Typically, an antibody specifically binds an antigen when it has a $K_d$ of at least about 1 $\mu$M or lower, more usually at least about 0.1 $\mu$M or lower, and preferably at least about 10 nM or lower for that antigen.

A variety of immunoassay formats (e.g., Western blots, ELISAs, etc.) may be used to select antibodies specifically immunoreactive with a particular protein. For example, solid-phase ELISA immunoassays are routinely used to select antibodies specifically immunoreactive with a protein (see, e.g., Harlow and Lane, Antibodies: A Laboratory Manual, New York: Cold Spring Harbor Press, (1990) for a description of immunoassay formats and conditions that can be used to determine specific immunoreactivity).

As used herein, the term "human antibody" means any antibody in which the variable and constant domain sequences are human sequences. The term encompasses antibodies with sequences derived from human genes, but which have been changed, e.g. to decrease possible immunogenicity, increase affinity, eliminate cysteines that might cause undesirable folding, etc. The term encompasses such antibodies produced recombinantly in non-human cells, which might impart glycosylation not typical of human cells. These antibodies may be prepared in a variety of ways, as described below.

The term "chimeric antibody" as used herein means an antibody that comprises regions from two or more different antibodies. In one embodiment, one or more of the CDRs are derived from a human anti-IL-6 antibody. In another embodiment, all of the CDRs are derived from a human anti-IL-6 antibody. In another embodiment, the CDRs from more than one human anti-IL-6 antibodies are combined in a chimeric antibody. For instance, a chimeric antibody may comprise a CDR1 from the light chain of a first human anti-IL-6 antibody, a CDR2 from the light chain of a second human anti-IL-6 antibody and a CDR3 from the light chain of a third human anti-IL-6 antibody, and the CDRs from the heavy chain may be derived from one or more other anti-IL-6 antibodies. Further, the framework regions may be derived from one of the anti-IL-6 antibodies from which one or more of the CDRs are taken or from one or more different human antibodies. For example, one or more CDRs from a non-human species (e.g., mouse or rat) antibody may be recombinantly inserted into a human antibody framework resulting in a "humanized" antibody.

DETAILED DESCRIPTION

The present invention relates to methods of treating a subject suffering from osteoarthritis by administering a therapeutically effective amount of an anti-IL-6 antibody. Methods have been described for generating IL-6 antibodies (see e.g., Wendling et al. (1993) J. Rheumatol. 20: 259-262; US Patent No. 5,618,700), including humanized anti-human IL-6 antibodies (see e.g., US Patent Nos. 6,121,423 and 5,856,135), and IL-6R antibodies (see e.g., U.S. Patent Nos. 5,795,965 and 5,817,790); MRA (tocilizumab; atilzumab; rhPM-1 (Drugs of the Future (2003) 28: 314-319) (Chugai Pharmaceutical Co., Ltd.) which was derived from the mouse anti-human IL-6R antibody PM1 (see e.g., Hirata et al. (1999) J. Immunol. 143: 2900-2906).

For preparation of IL-6 and IL-6R monoclonal or polyclonal antibodies, technique knowns in the art can be used (see, e.g., Kohler & Milstein, Nature 256:495-497 (1975); Kozbor et al., Immunology Today 4: 72 (1983); Cole et al., pp. 77-96 in Monoclonal Antibodies and Cancer Therapy, Alan R. Liss, Inc. (1985)). In addition, phage display technology can be used to identify single chain antibodies and heteromeric Fab fragments that specifically bind to selected antigens (see, e.g., McCafferty et al, Nature 348:552-554 (1990); Marks et al., Biotechnology 10:779-783 (1992)). Typically IL-6 and IL-6R polypeptides are employed to generate IL-6 and IL-6R antibodies, respectively. In the case of IL-6 polypeptides, they can be purified from native sources, cells that naturally secrete IL-6 polypeptides. Alternatively, synthetic peptides derived from IL-6 and IL-6R sequences disclosed herein and conjugated to a carrier protein can be used as an immunogen. In addition, recombinant IL-6 or IL-6R polypeptides can be employed to generate cognate antibodies. For example, recombinant mouse IL-6 (Catalog No. 406-ML-025), rat IL-6 (Catalog No. 506-RL-050) and human IL-6 (Catalog No. 206-IL-010) polypeptides as well as a recombinant soluble extracellular domain human IL-6R polypeptide (Catalog No. 227-SR-025) are commercially available from R&D Systems Inc., Minneapolis, MN. In addition, nucleic acids encoding IL-6 (see e.g., Hirano et al. (1986) Nature 324: 73-76; Brakenhoff et al. (1987) J. Immunol. 139: 4116-4121; SEQ ID NOS: 1, 3, and 5) and IL-6R (see e.g., Yamasaki et al. (1988) Science 241: 825-828; SEQ ID NOS: 7, 9, and 11) can be made or isolated using routine techniques in the field of recombinant genetics and synthetic nucleic acid chemistry. Basic texts disclosing the general methods of use in this invention include Sambrook et al., Molecular Cloning, A Laboratory Manual, 2nd ed., 1989; Kriegler, Gene Transfer and Expression: A Laboratory Manual, 1990; and Current Protocols in Molecular Biology, Ausubel et al., eds., 1998.

Polyclonal antibodies typically can be generated by immunization of an animal with the antigen of choice. The immunization of the animals can be by any method known in the art. See, e.g., Harlow and Lane, Antibodies: A Laboratory Manual, New York: Cold Spring Harbor Press, 1990. Methods for immunizing non-human animals such as mice, rabbits, rats, sheep, goats, pigs, cattle and horses are well known in the art. See, e.g., Harlow and Lane, *supra,* and U.S. Patent 5,994,619.

In certain embodiments, an IL-6 antigen is administered with an adjuvant to stimulate the immune response. Exemplary adjuvants include complete or incomplete Freund's adjuvant, RIBI (muramyl dipeptides) or ISCOM (immunostimulating complexes). Preferably, if a polypeptide is being administered, the immunization schedule will involve two or more administrations of the polypeptide, spread out over several weeks.

After immunization of an animal with an IL-6 or an IL-6R antigen, polyclonal antibodies and/or antibody-producing cells can be obtained from the animal. In some embodiments, anti-IL-6 or anti-IL-6R antibody-containing serum is obtained from the animal by bleeding or sacrificing the animal. The serum may be used as it is obtained from the animal, an immunoglobulin fraction may be obtained from the serum, or the anti-IL-6 or anti-IL-6R antibodies may be purified from the serum.

The animal's immune response to an immunogen preparation can be monitored by taking test bleeds and determining the titer of reactivity to the protein of choice. When appropriately high titers of antibody to the immunogen are obtained, blood can be collected from the animal and antisera are prepared. The level of IL-6 or IL-6R antibodies in serum can be assayed using an IL-6 or an IL-6R immunoassay. The polyclonal antibodies can be purified from the serum of an immunized animal using standard antibody and protein purification techniques.

Monoclonal antibodies can also be prepared against IL-6 and IL-6R. In certain embodiments, hybridoma techniques can be used to generate monoclonal antibodies. For example, antibody-producing immortalized cell lines can be prepared from cells isolated from the immunized animal. After immunization, the animal is sacrificed and lymph node and/or splenic B cells are immortalized. Methods of immortalizing cells include, but are not limited to, transfecting them with oncogenes, infecting them with an oncogenic virus, cultivating them under conditions that select for immortalized cells, subjecting them to carcinogenic or mutating compounds, fusing them with an immortalized cell, e.g., a myeloma cell, and inactivating a tumor suppressor gene. See, e.g., Harlow and Lane, *supra.* If fusion with myeloma cells is used, the myeloma cells preferably do not secrete immunoglobulin polypeptides (a non-secretory cell line).

Immortalized cells can be screened using IL-6 or IL-6R, or portions thereof, or a cell expressing IL-6 or IL-6R. In certain embodiments, the initial screening can be performed using an enzyme-linked immunoassay (ELISA) or a radioimmunoassay.

In some embodiments, human antibodies are produced by immunizing a non-human animal comprising in its genome some or all of human immunoglobulin heavy chain and light chain loci with an IL-6 or an IL-6R antigen. In certain embodiments, the non-human animal can be a XENOMOUSE™ animal (Abgenix Inc., Fremont, CA). Another non-human animal that may be used is a HuMAb-Mouse®, a transgenic mouse produced by Medarex (Medarex, Inc., Princeton, NJ).

XENOMOUSE™ mice are engineered mouse strains that comprise large fragments of human immunoglobulin heavy chain and light chain loci and are deficient in mouse antibody production. See, e.g., Green et al., Nature Genetics 7:13-21 (1994) and U.S. Patents 5,916,771, 5,939,598, 5,985,615, 5,998,209, 6,075,181, 6,091,001, 6,114,598, 6,130,364, 6,162,963 and 6,150,584. The splenic B cells from a XENOMOUSE™ can be fused to a non-secretory mouse myeloma (e.g, the myeloma cell line P3-X63-AG8-653) and monoclonal antibodies may be identified from the resulting pool of hybridomas. The IL-6 or IL-6R antibodies secreted by a hybridoma may be purified from a hybridoma culture and used in the methods of the present invention. The nucleic acids encoding the heavy and light chains of the IL-6 or IL-6R antibody may be isolated from a hybridoma and expressed in a host cell, e.g., NSO cells, CHO cells etc., to provide a source material from which purified IL-6 or IL-6 antibodies may be obtained.

In another embodiment, a transgenic animal is immunized with IL-6 or IL-6R, primary cells, e.g., spleen or peripheral blood cells, are isolated from an immunized transgenic animal and individual cells producing antibodies specific for the desired antigen are identified. Polyadenylated mRNA from each individual cell is isolated and reverse transcription polymerase chain reaction (RT-PCR) is performed using sense primers that anneal to variable region sequences, e.g., degenerate primers that recognize most or all of the FR1 regions of human heavy and light chain variable region genes and antisense primers that anneal to constant or joining region sequences. The cDNAs of the heavy and light chain variable regions are then cloned and expressed in any suitable host cell, e.g., a myeloma cell, as chimeric antibodies with respective immunoglobulin constant regions, such as the heavy chain and $\kappa$ or $\lambda$ constant

domains. See Babcook, J.S. et al., Proc. Natl. Acad. Sci. USA 93:7843-48, 1996. Anti IL-6 or IL-6R antibodies may then be identified and isolated as described herein.

In another aspect, the disclosure provides a method for making humanized anti-IL-6 or anti-IL-6R antibodies. In some embodiments, rats or mice are immunized with an IL-6 or an IL-6R antigen as described below under conditions that permit antibody production. Antibody-producing cells are isolated from the animals, fused with myelomas to produce hybridomas, and nucleic acids encoding the heavy and light chains of an anti-IL-6 or an anti-IL-6R antibody of interest are isolated. These nucleic acids are subsequently engineered using techniques known to those of skill in the art and as described further below to reduce the amount of non-human sequence, i.e., to humanize the antibody to reduce the immune response in humans

In another embodiment, phage display techniques can be used to provide libraries containing a repertoire of antibodies with varying affinities for IL-6 or IL-6R. By way of example, one method for preparing the library of antibodies for use in phage display techniques comprises the steps of immunizing a non-human animal comprising human immunoglobulin loci with an IL-6 or an IL-6R polypeptide to create an immune response, extracting antibody producing cells from the immunized animal; isolating RNA from the extracted cells, reverse transcribing the RNA to produce cDNA, amplifying the cDNA using a primer, and inserting the cDNA into a phage display vector such that antibodies are expressed on the phage. The resulting phage are tested for immunoreactivity to an IL-6 or IL-6R polypeptide. Recombinant anti-IL-6 or anti-IL-6R antibodies of the invention may be obtained in this way.

Techniques for the identification of high affinity human antibodies from such libraries are described for example in U.S. Patent No. 5,223,409; PCT Publication Nos. WO 92/18619, WO 91/17271, WO 92/20791, WO 92/15679, WO 93/01288, WO 92/01047, WO 92/09690; Fuchs et al., Bio/Technology 9:1370-1372 (1991); Hay et al., Hum. Antibod. Hybridomas 3:81-85 (1992); Huse et al., Science 246:1275-1281 (1989); McCafferty et al., Nature 348:552-554 (1990); Griffiths et al., EMBO J. 12:725-734 (1993); Hawkins et al., J. Mol. Biol. 226:889-896 (1992); Clackson et al., Nature 352:624-628 (1991); Gram et al., Proc. Natl. Acad. Sci. USA 89:3576-3580 (1992); Garrad et al., Bio/Technotogy 9:1373-1377 (1991); Hoogenboom et al., Nuc. Acid Res. 19:4133-4137 (1991); and Barbas et al., Proc. Natl. Acad. Sci. USA 88:7978-7982 (1991).

There are commercially available kits for generating phage display libraries (e.g., the Pharmacia Recombinant Phage Antibody System, catalog no. 27-9400-01; and the Stratagene SurfZAP™ phage display kit, catalog no. 240612) as well as commercially available systems for producing fully human phage expressed antibodies such as Cambridge Antibody Technology PLC (Cambridge, United Kingdom) and MorphoSys AG (e.g., HuCAL® GOLD technology, Martinsried, Germany).

Following screening and isolation of an anti-IL-6 or an anti-IL-6R antibody from a recombinant immunoglobulin display library, nucleic acids encoding the selected antibody can be recovered from the display package (e.g., from the phage genome) and subcloned into other expression vectors by standard recombinant DNA techniques. For example, the DNA encoding a phage expressed antibody can be cloned into a recombinant expression vector and introduced into a mammalian host cells or prokaryotic cells as appropriate for that antibody.

Pharmaceutical Compositions

The invention also relates to pharmaceutical compositions comprising an anti-IL-6 antibody for the treatment of subjects in need of treatment for osteoarthritis. Treatment may involve administration of one or more anti-IL-6 monoclonal antibodies of the invention, alone or with a pharmaceutically acceptable carrier. As used herein, "pharmaceutically acceptable carrier" means any and all solvents, dispersion media, coatings, antibacterial and antifungal agents, isotonic and absorption delaying agents, and the like that are physiologically compatible. Some examples of pharmaceutically acceptable carriers are water, saline, phosphate buffered saline, dextrose, glycerol, ethanol and the like, as well as combinations thereof. In many cases, isotonic agents, for example, sugars, polyalcohols such as mannitol, sorbitol, or sodium chloride can be present in the composition. Additional examples of pharmaceutically acceptable substances are wetting agents or minor amounts of auxiliary substances such as wetting or emulsifying agents, preservatives or buffers, which enhance the shelf life or effectiveness of the antibody.

The compositions of this invention may be in a variety of forms, for example, liquid, semi-solid and solid dosage forms, such as liquid solutions (e.g., injectable and infusible solutions), dispersions or suspensions, tablets, pills, powders, liposomes and suppositories. The particular form depends on the intended mode of administration and therapeutic application. Typical compositions are in the form of injectable or infusible solutions, such as compositions similar to those used for passive immunization of humans.

Therapeutic compositions typically are sterile and stable under the conditions of manufacture and storage. The composition can be formulated as a solution, microemulsion, dispersion, liposome, or other ordered structure suitable to high drug concentration. Sterile injectable solutions can be prepared by incorporating the anti-IL-6 antibody in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the methods of preparation include vacuum drying and freeze-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatin.

In certain embodiments, the antibody composition may be prepared with a carrier that will protect the antibody against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. See, e.g., Sustained and Controlled Release Drug Delivery Systems (J. R. Robinson, ed., Marcel Dekker, Inc., New York, 1978).

## Therapeutic Methods of Use

In another embodiment the invention provides for an anti-IL-6 antibody in the preparation of a medicament for the use of treating a subject suffering from osteoarthritis by administering a therapeutically effective amount of an anti-IL-6 antibody to a subject in need thereof A "therapeutically effective amount" refers to an amount, at dosages and for periods of time necessary, sufficient to inhibit, halt, or allow an improvement in the disorder or condition being treated when administered alone or in conjunction with another pharmaceutical agent or treatment in a particular subject or subject population. The term "subject" refers to a member of the class Mammalia. Examples of mammals include, without limitation, humans, primates, chimpanzees, rodents, mice, rats, rabbits, horses, dogs, cats, sheep, and cows. For example in a human or other mammal, a therapeutically effective amount can be determined experimentally in a laboratory or clinical setting, or may be the amount required by the guidelines of the United States Food and Drug Administration, or equivalent foreign agency, for the particular disease and subject being treated.

It should be appreciated that the determination of proper dosage forms, dosage amounts, and routes of administration is within the level of ordinary skill in the pharmaceutical and medical arts. A therapeutically effective amount of the antibody may vary according to factors such as the disease state, age, sex, and weight of the individual, and the ability of the antibody to elicit a desired response in the individual. A therapeutically effective amount is also one in which any toxic or detrimental effects of an agent are outweighed by the therapeutically beneficial effects.

The antibody may be administered once or multiple times. For example, the antibody may be administered from three times daily to once every six months or longer. The administering may be on a schedule such as three times daily, twice daily, once daily, once every two days, once every three days, once weekly, once every two weeks, once every month, once every two months, once every three months and once every six months.

Co-administration of an antibody with an additional therapeutic agent (combination therapy) encompasses administering a pharmaceutical composition comprising the anti-IL-6 antibody and the additional therapeutic agent and administering two or more separate pharmaceutical compositions, one comprising the anti-IL-6 antibody and the other(s) comprising the additional therapeutic agent(s). Further, co-administration or combination therapy refers to antibody and additional therapeutic agents being administered at the same time as one another, as wells as instances in which an antibody and additional therapeutic agents are administered at different times. For instance, an antibody may be administered once every three days, while the additional therapeutic agent is administered once daily. Alternatively, an antibody may be administered prior to or subsequent to treatment of the disorder with the additional therapeutic agent. An antibody and one or more additional therapeutic agents (the combination therapy) may be administered once, twice or at least the period of time until the condition is treated, palliated or cured.

For example, anti-IL-6 antibody may be co-administered with agents such as TNF-$\alpha$ antibodies such as REMICADE™, CDP-870 and HUMIRA™, TNF$\alpha$ receptor immunoglobulin fusion molecules (such as ENBREL™), COX-2 inhibitors (such as celecoxib, rofecoxib, parecoxib, valdecoxib, and etoricoxib), metalloprotease-13 inhibitors (preferably MMP-13 selective inhibitors), non-steroidal anti-inflammatory agents (" NSAIDs") such as piroxicam, diclofenac, propionic acids such as naproxen, flurbiprofen, fenoprofen, ketoprofen and ibuprofen, fenamates such as mefenamic acid, indomethacin, sulindac, apazone, pyrazolones such as phenylbutazone, salicylates such as aspirin, 6-(5-carboxy-5-methyl-hexyloxy)-2,2-dimethyl-hexanoic acid, calcium salt (gemcabene calcium), $\alpha 2\delta$ ligands (such as NEUROTIN™ AND PREGABALIN™), and intraarticular therapies such as corticosteroids and hyaluronic acids such as hyalgan and synvisc.

The antibodies of the present invention can be administered by a variety of methods known in the art including, via an oral, mucosal, buccal, intranasal, inhalable, intravenous, subcutaneous, intramuscular, parenteral, or topical route. In certain embodiments, the mode of administration is parenteral (e.g., intravenous, subcutaneous, intraperitoneal, intramuscular). In certain embodiments, the antibody is administered by intravenous infusion or injection. In particular embodiment, the antibody is administered by intrarticular, intramuscular or subcutaneous injection. As will be appreciated by the skilled artisan, the route and/or mode of administration will vary depending upon the desired results.

Dosage regimens can be adjusted to provide the optimum desired response (e.g., a therapeutic response). For example, a single bolus can be administered, several divided doses can be administered over time or the dose can be proportionally reduced or increased as indicated by the exigencies of the therapeutic situation. Parenteral compositions can be formulated in dosage unit form for ease of administration and uniformity of dosage. Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier

An exemplary, non-limiting range for a therapeutically effective amount of an antibody of the invention from 1 to 40 mg/kg. In certain embodiments, the dose is 8-20 mg. In other embodiments, the dose is 10-12 mg. In certain embodiments, a dose range for intrarticular injection would be a 15-30 mg/dose. It is to be noted that dosage values may vary with the type and severity of the condition to be alleviated. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the claimed composition.

Examples 1-3

MATERIALS and METHODS

Anti-IL-6 antibodies and anti-IL-6 receptor antibodies can be assayed for their ability to decrease quantitative or qualitative markers in in vivo models of osteoarthritis. For example, a monosodium iodoacetate-induced model of osteoarthritis (see e.g., Bove et al. (2003) Osteoarthritis and Cartilage 11: 821-830) can be carried out in rats to assess the effect of IL-6 antibodies in a weight bearing assay.

In Examples 1-3 on Day 0 rats are anesthetized with isofluorine, and the right, hind leg knee joint of a male Wistar rat is injected with 1.0 mg of mono-iodoacetate ("MIA") in 50 μl phosphate buffered saline (PBS) through the infrapatellar ligament and the left, hind leg knee joint is injected with 50 μlof saline through the infrapatellar ligament. The injection of MIA into the joint results in the inhibition of glycolysis and eventual death of surrounding chondrocytes. On the day before antibody administration, Day 6 or Day 13 post-MIA injection, the hind-paw weight differential between the arthritic right hind joint and the saline injected left hind joint of male Wistar rats (150 g) is determined with an incapacitance tester, model 2KG (Linton Instrumentation, Norfolk, United Kingdom). The incapacitance tester has a chamber on top with an outwardly sloping front wall that supports a rat's front limbs, and two weight sensing pads, one for each hind paw.

The rats are then further administered via intra-articular injection or intraperitoneally, with 50 μl PBS containing 1, 3, 10, 20, or 30 μg of either a polyclonal goat anti-rat IL-6 antibody (R&D Systems Inc., Minneapolis, MN), or a polyclonal anti-rat IgG antibody (Product No. R 5005, Sigma, St. Louis, MO) on day 7 or day 14 post MIA-injection and the hind-paw weight differential is measured at 0-24 hours post antibody injection.

The percent inhibition of a change in hind paw joint function is calculated as the percent change in hind-paw weight distribution for treated animals versus control animals at the same time point (e.g., polyclonal anti-IL-6 antibody versus polyclonal anti-IgG antibody at 2 hours post injection). For example,

$$\text{Percent inhibition of a change in hind paw weight distribution} = \left\{ 1 - \left[ \frac{(\Delta W_G)}{(\Delta W_C)} \right] \right\} \times 100$$

wherein:

$\Delta W_C$ is the hind-paw weight differential between the healthy left limb and the arthritic limb of the control animal administered the anti-rat IgG antibody alone, as measured at a particular time point (e.g., 1, 4, or 24 hours) post injection Day 7 or Day 14; and

$\Delta W_G$ is the hind-paw weight differential between the healthy left limb and the arthritic limb of the animal administered the anti-rat IL-6 antibody, as measured at the same time point used to determine $\Delta W_C$.

EXAMPLE 1

The MIA model was carried out as described above under Materials and Methods, as follows: rats were induced with MIA as described above, and administered 1, 3, 10, 20, or 30 μg of the polyclonal IL-6 antibody or the polyclonal IgG antibody in the right arthritic knee in a 50 μlvolume of PBS and 50 μl volume of PBS in the left control knee on day 7 post-MIA injection. Six rats were injected at each dose. After one-hour post-antibody injection, the weight differential was measured. The percent inhibition of a change in hind paw weight distribution of the IL-6 antibody treated rats as compared to the polyclonal IgG antibody treated rats is reported in Table 1. The 20 and 30 microgram doses of IL-6 antibody significantly inhibited ($p<0.05$) the change in hind paw weight distribution versus polyclonal rat IgG. Data are presented as the mean percent inhibition ± standard error of the mean (SEM).

Table 1

| Dose (μg/knee) | % Inhibition |
|---|---|
| 1 | 28±5 |
| 3 | 27±12 |
| 10 | 18±8 |
| 20 | 60±7* |
| 30 | 63±4* |
| *p<0.05 vs. polyclonal rat IgG (One-Factor ANCOVA followed by Hochberg's procedure) | |

EXAMPLE 2

The MIA model was carried out as described above under Materials and Methods, as follows: rats were induced with MIA as described above, and administered 30 μg of the IL-6 antibody in the right arthritic knee in a 50 μl volume of PBS and 50 μl volume of PBS in the left control knee on day 14 post-MIA injection. Eight rats were injected at each dose. After one hour, 4 hours, and 24 hours post-antibody injection, the weight differential was measured and reported as the mean ± the standard error of the mean in Table 2. The 30 microgram dose of IL-6 antibody significantly decreased ($p<0.05$) the change in hind paw weight distribution at 1, 4, and 24 hours versus time zero (pre-antibody injection).

Table 2

| Time post-injection of antibody (hours) | Weight differential (grams) (Mean $\Delta W_G$ ± SEM) |
|---|---|
| 0 | 33 ±3 |
| 1 | 17±2* |
| 4 | 19±2* |
| 24 | 17 ± 1* |
| *p<0.05 vs. time zero (paired t-test followed by Hochberg's procedure) | |

EXAMPLE 3

The MIA model was carried out as described above under Materials and Methods, as follows: rats were induced with MIA as described above, and administered 30 μg of the IL-6 antibody via an intraperitoneal injection in a 50 μl volume of PBS on day 14 post-MIA injection. Eight rats were injected at each dose. The weight differential ($\Delta W_G$)was measured and reported as the mean $\Delta W_G$ ± the standard error of the mean in Table 3 for the time points of just prior to antibody injection, at one hour, and at 4 hours post-antibody injection. The 30 microgram dose of IL-6 antibody did not significantly inhibit the change in hind paw weight distribution versus time zero (pre-antibody injection).

Table 3

| Time post-injection of antibody (hours) | Weight differential (grams) (Mean $AW_G$ ± SEM) |
|---|---|
| 0 | 32±2 |
| 1 | 28±1 |
| 4 | 32±2 |

## Claims

1. Use of an anti-IL-6 antibody in the preparation of a medicament for the treatment of osteoarthritis in mammals.

2. Use, according to claim 1, wherein said medicament is a pharmaceutical composition comprising a pharmaceutically acceptable carrier and a therapeutically effective amount of an anti-IL6 antibody.

3. Use according to claim 1 or 2, wherein said medicament is adapted to be administered interarticularly or intravenously.

4. Use according to claim 1 or 2, wherein said agent is a monoclonal IL-6 antibody.

5. Use according to claim 4, wherein said IL-6 antibody is an anti-human IL-6 antibody.

6. Use according to claim 4 or 5, wherein said medicament composition is adapted to be administered interarticularly.

7. Use according to claim 4 or 5, wherein said medicament is adapted to be administered intravenously.

8. Use according to any of the preceding claims, wherein said medicament is adapted to be administered with one or more agents selected from the group consisting of: 6-(5-carboxy-5-methyl-hexyloxy)- 2,2-dimethyl-hexanoicacid calcium salt, non-steroidal anti-inflammatory agents, piroxicam, diclofenac, naproxen, flurbiprofen, fenoprofen, ketoprofen, ibuprofen, mefenamic acid, indomethacin, sulindac, apazone, phenylbutazone, aspirin™, corticosteroids, hyalgan, and synvisc™.

9. Use according to any of the preceding claims, wherein said medicament is adapted to be administered with one or more agents selected from the group consisting of: parecoxib, celecoxib, valdecoxib, and etoricoxib.

10. An anti-IL-6 antibody for use in the treatment of osteoarthritis in mammals

## Patentansprüche

1. Verwendung eines Anti-IL-6-Antikörpers bei der Herstellung eines Medikaments für die Behandlung von Osteoarthritis bei Säugern.

2. Verwendung gemäß Anspruch 1, wobei das Medikament eine pharmazeutische Zusammensetzung ist, die einen pharmazeutisch verträgliche Träger und eine therapeutisch wirksame Menge eines Anti-IL-6-Antikörpers umfasst.

3. Verwendung gemäß Anspruch 1 oder 2, wobei das Medikament angepasst ist, um interartikulär oder intravenös verabreicht zu werden.

4. Verwendung gemäß Anspruch 1 oder 2, wobei das Agens ein monoklonaler IL-6-Antikörper ist.

5. Verwendung gemäß Anspruch 4, wobei der IL-6-Antikörper ein Anti-Human-IL-6-Antikörper ist.

6. Verwendung gemäß Anspruch 4 oder 5, wobei die Medikamentenzusammensetzung angepasst ist, um interartikulär verabreicht zu werden.

7. Verwendung gemäß Anspruch 4 oder 5, wobei das Medikament angepasst ist, um intravenös verabreicht zu werden.

8. Verwendung gemäß einem der vorangehenden Ansprüche, wobei das Medikament angepasst ist, um mit einem Agens oder mehreren Agentien, ausgewählt aus der Gruppe, bestehend aus: 6-(5-Carboxy-5-methyl-hexyloxy)-2,2-dimethylhexansäure-calciumsalz, nicht-steroidalen Antirheumatika, Piroxicam, Diclofenac, Naproxen, Flurbiprofen, Fenoprofen, Ketoprofen, Ibuprofen, Mefenaminsäure, Indomethacin, Sulindac, Apazon, Phenylbutazon, Aspirin™, Corticosteroiden, Hyalagan und Synvisc™, verabreicht zu werden.

9. Verwendung gemäß einem der vorangehenden Ansprüche, wobei das Medikament angepasst ist, um mit einem Agens oder mehreren Agenzien, ausgewählt aus der Gruppe, bestehend aus Parecoxib, Celecoxib, Valdecoxib und Etoricoxib, verabreicht zu werden.

**10.** Anti-IL-6-Antikörper zur Verwendung bei der Behandlung von Osteoarthritis bei Säugern.

**Revendications**

**1.** Utilisation d'un anticorps anti-IL-6 dans la préparation d'un médicament pour le traitement de l'arthrose chez des mammifères.

**2.** Utilisation selon la revendication 1, dans laquelle ledit médicament est une composition pharmaceutique comprenant un support pharmaceutiquement acceptable et une quantité thérapeutiquement efficace d'un anticorps anti-IL-6.

**3.** Utilisation selon la revendication 1 ou 2, dans laquelle ledit médicament est adapté à une administration interarticulaire ou intraveineuse.

**4.** Utilisation selon la revendication 1 ou 2, dans laquelle ledit agent est un anticorps de IL-6 monoclonal.

**5.** Utilisation selon la revendication 4, dans laquelle ledit anticorps de IL-6 est un anticorps anti-IL-6 humaine.

**6.** Utilisation selon la revendication 4 ou 5, dans laquelle ladite composition de médicament est adaptée à une administration interarticulaire.

**7.** Utilisation selon la revendication 4 ou 5, dans laquelle ledit médicament est adapté à une administration intraveineuse.

**8.** Utilisation selon l'une quelconque des revendications précédentes, dans laquelle ledit médicament est adapté à une administration avec un ou plusieurs agents sélectionnés dans le groupe consistant en : le sel de calcium de l'acide 6-(5-carboxy-5-méthyl-hexyloxy)-2,2-diméthyl-hexanoïque, les agents anti-inflammatoires non stéroïdiens, le piroxicam, le diclofénac, le naproxène, le flurbiprofène, le fénoprofène, le kétoprofène, l'ibuprofène, l'acide méfénamique, l'indométhacine, le sulindac, l'apazone, la phénylbutazone, l'aspirine™, les corticostéroïdes, le hyalgan et le synvisc™.

**9.** Utilisation selon l'une quelconque des revendications précédentes, dans laquelle ledit médicament est adapté pour être administré avec un ou plusieurs agents sélectionnés dans le groupe consistant en : le parécoxib, le célécoxib, le valdécoxib et l'étoricoxib.

**10.** Anticorps anti-IL-6 pour une utilisation dans le traitement de l'arthrose chez des mammifères.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0331640 A2
- US 5618700 A
- US 6121423 A
- US 5856135 A
- US 5795965 A
- US 5817790 A
- US 5994619 A
- US 5916771 A
- US 5939598 A
- US 5985615 A
- US 5998209 A
- US 6075181 A
- US 6091001 A
- US 6114598 A
- US 6130364 A
- US 6162963 A
- US 6150584 A
- US 5223409 A
- WO 9218619 A
- WO 9117271 A
- WO 9220791 A
- WO 9215679 A
- WO 9301288 A
- WO 9201047 A
- WO 9209690 A
- US 60543814 A
- US 60543814 B
- WO IB2005000240 W


### Non-patent literature cited in the description


- **CREAMER et al.** *J. Rheumatol.,* 1999, vol. 26, 1785-1792
- **WARD et al.** *Nature,* 1989, vol. 341, 544-546
- **BIRD et al.** *Science,* 1988, vol. 242, 423-426
- **HUSTON et al.** *Proc. Natl. Acad. Sci. USA,* 1988, vol. 85, 5879-5883
- **HOLLIGER P. et al.** *Proc. Natl. Acad. Sci. USA,* 1993, vol. 90, 6444-6448
- **POLJAK R. J. et al.** *Structure,* 1994, vol. 2, 1121-1123
- **VAN ZAANEN et al.** *Br. J. Haematol.,* 1998, vol. 102, 783-790
- **HARLOW ; LANE.** Antibodies: A Laboratory Manual. Cold Spring Harbor Press, 1990
- **WENDLING et al.** *J. Rheumatol.,* 1993, vol. 20, 259-262
- *Drugs of the Future,* 2003, vol. 28, 314-319
- **HIRATA et al.** *J. Immunol.,* 1999, vol. 143, 2900-2906
- **KOHLER ; MILSTEIN.** *Nature,* 1975, vol. 256, 495-497
- **KOZBOR et al.** *Immunology Today,* 1983, vol. 4, 72
- **COLE et al.** Monoclonal Antibodies and Cancer Therapy. Alan R. Liss, Inc, 1985, 77-96
- **MCCAFFERTY et al.** *Nature,* 1990, vol. 348, 552-554
- **MARKS et al.** *Biotechnology,* 1992, vol. 10, 779-783
- **HIRANO et al.** *Nature,* 1986, vol. 324, 73-76
- **BRAKENHOFF et al.** *J. Immunol.,* 1987, vol. 139, 4116-4121
- **YAMASAKI et al.** *Science,* 1988, vol. 241, 825-828
- **SAMBROOK et al.** Molecular Cloning, A Laboratory Manual. 1989
- **KRIEGLER.** Gene Transfer and Expression: A Laboratory Manual. 1990
- Current Protocols in Molecular Biology. 1998
- **GREEN et al.** *Nature Genetics,* 1994, vol. 7, 13-21
- **BABCOOK, J.S. et al.** *Proc. Natl. Acad. Sci. USA,* 1996, vol. 93, 7843-48
- **FUCHS et al.** *Bio/Technology,* 1991, vol. 9, 1370-1372
- **HAY et al.** *Hum. Antibod. Hybridomas,* 1992, vol. 3, 81-85
- **HUSE et al.** *Science,* 1989, vol. 246, 1275-1281
- **GRIFFITHS et al.** *EMBO J.,* 1993, vol. 12, 725-734
- **HAWKINS et al.** *J. Mol. Biol.,* 1992, vol. 226, 889-896
- **CLACKSON et al.** *Nature,* 1991, vol. 352, 624-628
- **GRAM et al.** *Proc. Natl. Acad. Sci. USA,* 1992, vol. 89, 3576-3580
- **GARRAD et al.** *Bio/Technotogy,* 1991, vol. 9, 1373-1377
- **HOOGENBOOM et al.** *Nuc. Acid Res.,* 1991, vol. 19, 4133-4137

- **BARBAS et al.** *Proc. Natl. Acad. Sci. USA,* 1991, vol. 88, 7978-7982
- Sustained and Controlled Release Drug Delivery Systems. Marcel Dekker, Inc, 1978
- **BOVE et al.** *Osteoarthritis and Cartilage,* 2003, vol. 11, 821-830

SEQUENCE LISTING

<110> warner-Lambert Company LLC
Bove, Susan R
Kilgore, Kenneth

<120> Methods of Treating Osteoarthritis with IL-6 Antagonists

<130> PC32145A

<150> 60/543,814
<151> 2004-02-11

<160> 12

<170> PatentIn version 3.3

<210> 1
<211> 636
<212> DNA
<213> Mus musculus

<400> 1

```
atgaagttcc tctctgcaag agacttccat ccagttgcct tcttgggact gatgctggtg      60
acaaccacgg ccttccctac ttcacaagtc cggagaggag acttcacaga ggataccact     120
cccaacagac ctgtctatac cacttcacaa gtcggaggct taattacaca tgttctctgg     180
gaaatcgtgg aaatgagaaa agagttgtgc aatggcaatt ctgattgtat gaacaacgat     240
gatgcacttg cagaaaacaa tctgaaactt ccagagatac aaagaaatga tggatgctac     300
caaactggat ataatcagga aatttgccta ttgaaaattt cctctggtct tctggagtac     360
catagctacc tggagtacat gaagaacaac ttaaaagata acaagaaaga caaagccaga     420
gtccttcaga gagatacaga aactctaatt catatcttca accaagaggt aaaagattta     480
cataaaatag tccttcctac cccaatttcc aatgctctcc taacagataa gctggagtca     540
cagaaggagt ggctaaggac caagaccatc caattcatct tgaaatcact tgaagaattt     600
ctaaaagtca ctttgagatc tactcggcaa acctag                               636
```

<210> 2
<211> 211
<212> PRT
<213> Mus musculus

<400> 2

```
Met Lys Phe Leu Ser Ala Arg Asp Phe His Pro Val Ala Phe Leu Gly
1               5                   10                  15

Leu Met Leu Val Thr Thr Thr Ala Phe Pro Thr Ser Gln Val Arg Arg
            20                  25                  30

Gly Asp Phe Thr Glu Asp Thr Thr Pro Asn Arg Pro Val Tyr Thr Thr
        35                  40                  45

Ser Gln Val Gly Gly Leu Ile Thr His Val Leu Trp Glu Ile Val Glu
    50                  55                  60

Met Arg Lys Glu Leu Cys Asn Gly Asn Ser Asp Cys Met Asn Asn Asp
65                  70                  75                  80

Asp Ala Leu Ala Glu Asn Asn Leu Lys Leu Pro Glu Ile Gln Arg Asn
                85                  90                  95

Asp Gly Cys Tyr Gln Thr Gly Tyr Asn Gln Glu Ile Cys Leu Leu Lys
            100                 105                 110

Ile Ser Ser Gly Leu Leu Glu Tyr His Ser Tyr Leu Glu Tyr Met Lys
        115                 120                 125

Asn Asn Leu Lys Asp Asn Lys Lys Asp Lys Ala Arg Val Leu Gln Arg
    130                 135                 140

Asp Thr Glu Thr Leu Ile His Ile Phe Asn Gln Glu Val Lys Asp Leu
145                 150                 155                 160

His Lys Ile Val Leu Pro Thr Pro Ile Ser Asn Ala Leu Leu Thr Asp
                165                 170                 175

Lys Leu Glu Ser Gln Lys Glu Trp Leu Arg Thr Lys Thr Ile Gln Phe
            180                 185                 190

Ile Leu Lys Ser Leu Glu Glu Phe Leu Lys Val Thr Leu Arg Ser Thr
        195                 200                 205

Arg Gln Thr
    210
```

<210> 3
<211> 636

<212> DNA
<213> Rattus norvegicus

<400> 3

atgaagtttc tctccgcaag agacttccag ccagttgcct tcttgggact gatgttgttg 60
acagccactg ccttccctac ttcacaagtc cggagaggag acttcacaga ggataccacc 120
cacaacagac cagtatatac cacttcacaa gtcggaggct taattacata tgttctcagg 180
gagatcttgg aaatgagaaa agagttgtgc aatggcaatt ctgattgtat gaacagcgat 240
gatgcactgt cagaaaacaa tctgaaactt ccagaaatac aaagaaatga tggatgcttc 300
caaactggat ataaccagga aatttgccta ttgaaaatct gctctggtct tctggagttc 360

cgtttctacc tggagtttgt gaagaacaac ttacaagata acaagaaaga caaagccaga 420
gtcattcaga gcaatactga aaccctagtt catatcttca aacaagagat aaaagactca 480
tataaaatag tccttcctac cccaacttcc aatgctctcc taatggagaa gttagagtca 540
cagaaggagt ggctaaggac caagaccatc caactcatct tgaaagcact tgaagaattt 600
ctaaaggtca ctatgaggtc tactcggcaa acctag 636

<210> 4
<211> 211
<212> PRT
<213> Rattus norvegicus

<400> 4

Met Lys Phe Leu Ser Ala Arg Asp Phe Gln Pro Val Ala Phe Leu Gly
1 5 10 15

Leu Met Leu Leu Thr Ala Thr Ala Phe Pro Thr Ser Gln Val Arg Arg
20 25 30

Gly Asp Phe Thr Glu Asp Thr Thr His Asn Arg Pro Val Tyr Thr Thr
35 40 45

Ser Gln Val Gly Gly Leu Ile Thr Tyr Val Leu Arg Glu Ile Leu Glu
50 55 60

Met Arg Lys Glu Leu Cys Asn Gly Asn Ser Asp Cys Met Asn Ser Asp
65 70 75 80

Asp Ala Leu Ser Glu Asn Asn Leu Lys Leu Pro Glu Ile Gln Arg Asn
85 90 95

Asp Gly Cys Phe Gln Thr Gly Tyr Asn Gln Glu Ile Cys Leu Leu Lys
100 105 110

Ile Cys Ser Gly Leu Leu Glu Phe Arg Phe Tyr Leu Glu Phe Val Lys
115 120 125

Asn Asn Leu Gln Asp Asn Lys Lys Asp Lys Ala Arg Val Ile Gln Ser
130 135 140

Asn Thr Glu Thr Leu Val His Ile Phe Lys Gln Glu Ile Lys Asp Ser
145 150 155 160

Tyr Lys Ile Val Leu Pro Thr Pro Thr Ser Asn Ala Leu Leu Met Glu
165 170 175

Lys Leu Glu Ser Gln Lys Glu Trp Leu Arg Thr Lys Thr Ile Gln Leu
180 185 190

Ile Leu Lys Ala Leu Glu Glu Phe Leu Lys Val Thr Met Arg Ser Thr
195 200 205

Arg Gln Thr
210

<210> 5
<211> 639
<212> DNA
<213> Human

<400> 5

```
atgaactcct tctccacaag cgccttcggt ccagttgcct tctccctggg gctgctcctg      60
gtgttgcctg ctgccttccc tgccccagta cccccaggag aagattccaa agatgtagcc     120
gccccacaca gacagccact cacctcttca gaacgaattg acaaacaaat tcggtacatc     180
ctcgacggca tctcagccct gagaaaggag acatgtaaca agagtaacat gtgtgaaagc     240
agcaaagagg cactggcaga aaacaacctg aaccttccaa agatggctga aaaagatgga     300
tgcttccaat ctggattcaa tgaggagact tgcctggtga aaatcatcac tggtcttttg     360
gagtttgagg tatacctaga gtacctccag aacagatttg agagtagtga ggaacaagcc     420
agagctgtgc agatgagtac aaaagtcctg atccagttcc tgcagaaaaa ggcaaagaat     480
ctagatgcaa taaccacccc tgacccaacc acaaatgcca gcctgctgac gaagctgcag     540
gcacagaacc agtggctgca ggacatgaca actcatctca ttctgcgcag ctttaaggag     600
ttcctgcagt ccagcctgag ggctcttcgg caaatgtag                            639
```

<210> 6
<211> 212
<212> PRT
<213> Human

<400> 6

```
Met Asn Ser Phe Ser Thr Ser Ala Phe Gly Pro Val Ala Phe Ser Leu
1               5                   10                  15

Gly Leu Leu Leu Val Leu Pro Ala Ala Phe Pro Ala Pro Val Pro Pro
            20                  25                  30

Gly Glu Asp Ser Lys Asp Val Ala Ala Pro His Arg Gln Pro Leu Thr
        35                  40                  45

Ser Ser Glu Arg Ile Asp Lys Gln Ile Arg Tyr Ile Leu Asp Gly Ile
    50                  55                  60
```

```
Ser Ala Leu Arg Lys Glu Thr Cys Asn Lys Ser Asn Met Cys Glu Ser
65                  70                  75                  80

Ser Lys Glu Ala Leu Ala Glu Asn Asn Leu Asn Leu Pro Lys Met Ala
                85                  90                  95

Glu Lys Asp Gly Cys Phe Gln Ser Gly Phe Asn Glu Glu Thr Cys Leu
            100                 105                 110

Val Lys Ile Ile Thr Gly Leu Leu Glu Phe Glu Val Tyr Leu Glu Tyr
        115                 120                 125

Leu Gln Asn Arg Phe Glu Ser Ser Glu Glu Gln Ala Arg Ala Val Gln
    130                 135                 140

Met Ser Thr Lys Val Leu Ile Gln Phe Leu Gln Lys Lys Ala Lys Asn
145                 150                 155                 160

Leu Asp Ala Ile Thr Thr Pro Asp Pro Thr Thr Asn Ala Ser Leu Leu
                165                 170                 175

Thr Lys Leu Gln Ala Gln Asn Gln Trp Leu Gln Asp Met Thr Thr His
            180                 185                 190

Leu Ile Leu Arg Ser Phe Lys Glu Phe Leu Gln Ser Ser Leu Arg Ala
        195                 200                 205

Leu Arg Gln Met
    210
```

<210> 7
<211> 1323
<212> DNA
<213> Mus musculus

<400> 7

```
atgctgaccg tcggctgcac gctgttggtc gccctgctgg ccgcgcccgc ggtcgcgctg     60
gtcctcggga gctgccgcgc gctggaggtg gcaaatggca cagtgacaag cctgccaggg    120
gccaccgtta ccctgatttg ccccgggaag gaagcagcag gcaatgttac cattcactgg    180
gtgtactctg gctcacaaaa cagagaatgg actaccacag gaaacacact ggttctgagg    240
gacgtgcagc tcagcgacac tggggactat ttatgctccc tgaatgatca cctggtgggg    300
actgtgccct tgctggtgga tgttccccca gaggagccca agctctcctg cttccggaag    360
aaccccttg tcaacgccat ctgtgagtgg cgtccgagca gcaccccctc tccaaccacg    420
aaggctgtgc tgtttgcaaa gaaaatcaac accaccaacg ggaagagtga cttccaggtg    480
```

ccctgccagt attctcagca gctgaaaagc ttctcctgcc aggtggagat cctggagggt 540
gacaaagtat accacatagt gtcactgtgc gttgcaaaca gtgtgggaag caagtccagc 600
cacaacgaag cgtttcacag cttaaaaatg gtgcagccgg atccacctgc caaccttgtg 660
gtatcagcca tacctggaag gccgcgctgg ctcaaagtca gctggcagca ccctgagacc 720
tgggacccga gttactactt gctgcagttc cagcttcgat accgacctgt atggtcaaag 780
gagttcacgg tgttgctgct cccggtggcc cagtaccaat gcgtcatcca tgatgccttg 840
cgaggagtga agcacgtggt ccaggtccgt gggaaggagg agcttgacct tggccagtgg 900
agtgaatggt ccccagaggt cacgggcact ccttggatag cagagcccag gaccaccccg 960
gcaggaatcc tctggaaccc cacacaggtc tctgttgaag actctgccaa ccacgaggat 1020
cagtacgaaa gttctacaga agcaacgagt gtcctcgccc cagtgcaaga atcctcgtcc 1080
atgtccctgc ccacattcct ggtagctgga ggaagcttgg cgtttgggtt gcttctctgt 1140
gtcttcatca tcctgtgttg ggagccgcgc ccacattcgc cgttacaaga tggcgctgac 1200
agctgtgttc taagtggtaa acaaataatc tgcgcatgtg ccgagggtgg ttctccactc 1260
catgtgctct gccttccccg tgacgtcaac tcggccgatg ggctgcagcc aatcagggag 1320
tga 1323

<210> 8
<211> 364
<212> PRT
<213> Mus musculus

<400> 8

Met Leu Thr Val Gly Cys Thr Leu Leu Val Ala Leu Leu Ala Ala Pro
1 5 10 15

Ala Val Ala Leu Val Leu Gly Ser Cys Arg Ala Leu Glu Val Ala Asn
20 25 30

Gly Thr Val Thr Ser Leu Pro Gly Ala Thr Val Thr Leu Ile Cys Pro
35 40 45

Gly Lys Glu Ala Ala Gly Asn Val Thr Ile His Trp Val Tyr Ser Gly
50 55 60

Ser Gln Asn Arg Glu Trp Thr Thr Thr Gly Asn Thr Leu Val Leu Arg
65 70 75 80

Asp Val Gln Leu Ser Asp Thr Gly Asp Tyr Leu Cys Ser Leu Asn Asp
85 90 95

His Leu Val Gly Thr Val Pro Leu Leu Val Asp Val Pro Pro Glu Glu

100 105 110

Pro Lys Leu Ser Cys Phe Arg Lys Asn Pro Leu Val Asn Ala Ile Cys
115 120 125

Glu Trp Arg Pro Ser Ser Thr Pro Ser Pro Thr Thr Lys Ala Val Leu
130 135 140

Phe Ala Lys Lys Ile Asn Thr Thr Asn Gly Lys Ser Asp Phe Gln Val
145 150 155 160

Pro Cys Gln Tyr Ser Gln Gln Leu Lys Ser Phe Ser Cys Gln Val Glu
165 170 175

Ile Leu Glu Gly Asp Lys Val Tyr His Ile Val Ser Leu Cys Val Ala
180 185 190

Asn Ser Val Gly Ser Lys Ser Ser His Asn Glu Ala Phe His Ser Leu
195 200 205

Lys Met Val Gln Pro Asp Pro Pro Ala Asn Leu Val Val Ser Ala Ile
210 215 220

Pro Gly Arg Pro Arg Trp Leu Lys Val Ser Trp Gln His Pro Glu Thr
225 230 235 240

Trp Asp Pro Ser Tyr Tyr Leu Leu Gln Phe Gln Leu Arg Tyr Arg Pro
245 250 255

Val Trp Ser Lys Glu Phe Thr Val Leu Leu Leu Pro Val Ala Gln Tyr
260 265 270

Gln Cys Val Ile His Asp Ala Leu Arg Gly Val Lys His Val Val Gln
275 280 285

Val Arg Gly Lys Glu Glu Leu Asp Leu Gly Gln Trp Ser Glu Trp Ser
290 295 300

Pro Glu Val Thr Gly Thr Pro Trp Ile Ala Glu Pro Arg Thr Thr Pro
305 310 315 320

Ala Gly Ile Leu Trp Asn Pro Thr Gln Val Ser Val Glu Asp Ser Ala
325 330 335

Asn His Glu Asp Gln Tyr Glu Ser Ser Thr Glu Ala Thr Ser Val Leu
340 345 350

Ala Pro Val Gln Glu Ser Ser Ser Met Ser Leu Pro
355 360

<210> 9
<211> 1389
<212> DNA
<213> Rattus norvegicus

<400> 9

atgctggccg tcggctgcac cctgctggtc gccctgctgg ccgcgcccgc agtcgcgctg 60
gtccttggga gctgccgcgc gctggaggtg gcaaatggta cggtgacgag cctgccaggg 120
gccactgtta ccctgatctg ccctgggaag gaagcagcag gcaatgctac cattcactgg 180
gtgtactcag gctcacagag cagagaatgg actaccacgg gaaacacact ggttctgagg 240
gccgtgcagg tcaatgacac tgggcactat ttgtgcttcc tggatgatca tctggttggg 300
actgtgccct tgctggtgga tgttcccca gaggagccca agctctcctg cttccggaag 360
aacccccttg taaatgcctt ttgtgagtgg catccaagca gcactccctc tccaaccacg 420
aaggctgtga tgtttgcaaa gaaaatcaac accaccaatg ggaagagtga cttccaggtg 480
ccttgccagt attctcagca gctgaaaagc ttctcctgcg aggtggagat cctggagggt 540
gacaaagtgt accacatagt gtcactgtgc gttgcaaaca gtgtcggaag caggtccagc 600
cacaatgtag tatttcagag tttaaaaatg gtgcagccgg atccacctgc caaccttgtg 660
gtatcagcca tacctggaag cctcgttggc tcaaagtcag ttggcaagac cctgagtcct 720
gggacccaag ttactacttg ttgcaattcg agcttcgata ccgacctgta tggtcaaaga 780
acgttcacgg tgtggccgct ccaggtggcc cagcatcaat gtgtcatcca tgatgccttg 840
cgaggagtaa agcatgtggt gcaggtccga gggaaggagg agtttgacat tggccagtgg 900
agcaaatggt ccccggaggt cacaggcact ccttggctag cagagcccag gaccactccg 960
gcagggatcc cggggaaccc cacacaggtc tctgttgaag actatgacaa ccacgaggat 1020
cagtacggaa gttctacaga agcaacgagt gtcctcgccc cagtgcaagg atcctcgcct 1080
atacccctgc ccacattcct ggtagctgga ggaagcctgg cgtttggatt gcttctctgt 1140
gtcttcatca tcttgagact caagaagaaa tggaagtcac aggctgagaa ggaaagcaag 1200
acgacttctc cccaccgta tcccttggga ccgctgaagc cgaccttcct cctggttcct 1260
ctcctcaccc catcagggtc ccataacagc tctgggactg acaacaccgg aagccacagc 1320
tgcctgggtg tcagggaccc acagtgccct aatgacaaca gcaacagaga ctacttattc 1380
cccagataa 1389

<210> 10
<211> 364
<212> PRT
<213> Rattus norvegicus

<400> 10

Met Leu Ala Val Gly Cys Thr Leu Leu Val Ala Leu Leu Ala Ala Pro
1               5                   10                  15

Ala Val Ala Leu Val Leu Gly Ser Cys Arg Ala Leu Glu Val Ala Asn
            20                  25                  30

Gly Thr Val Thr Ser Leu Pro Gly Ala Thr Val Thr Leu Ile Cys Pro
        35                  40                  45

Gly Lys Glu Ala Ala Gly Asn Ala Thr Ile His Trp Val Tyr Ser Gly
    50                  55                  60

Ser Gln Ser Arg Glu Trp Thr Thr Thr Gly Asn Thr Leu Val Leu Arg
65                  70                  75                  80

Ala Val Gln Val Asn Asp Thr Gly His Tyr Leu Cys Phe Leu Asp Asp
                85                  90                  95

His Leu Val Gly Thr Val Pro Leu Leu Val Asp Val Pro Pro Glu Glu
            100                 105                 110

Pro Lys Leu Ser Cys Phe Arg Lys Asn Pro Leu Val Asn Ala Phe Cys
        115                 120                 125

Glu Trp His Pro Ser Ser Thr Pro Ser Pro Thr Thr Lys Ala Val Met
    130                 135                 140

Phe Ala Lys Lys Ile Asn Thr Thr Asn Gly Lys Ser Asp Phe Gln Val
145                 150                 155                 160

Pro Cys Gln Tyr Ser Gln Gln Leu Lys Ser Phe Ser Cys Glu Val Glu
                165                 170                 175

Ile Leu Glu Gly Asp Lys Val Tyr His Ile Val Ser Leu Cys Val Ala
            180                 185                 190

Asn Ser Val Gly Ser Arg Ser Ser His Asn Val Val Phe Gln Ser Leu
        195                 200                 205

Lys Met Val Gln Pro Asp Pro Pro Ala Asn Leu Val Val Ser Ala Ile
    210                 215                 220

Pro Gly Ser Leu Val Gly Ser Lys Ser Val Gly Lys Thr Leu Ser Pro
225                 230                 235                 240

Gly Thr Gln Val Thr Thr Cys Cys Asn Ser Ser Phe Asp Thr Asp Leu
245 250 255

Tyr Gly Gln Arg Thr Phe Thr Val Trp Pro Leu Gln Val Ala Gln His
260 265 270

Gln Cys Val Ile His Asp Ala Leu Arg Gly Val Lys His Val Val Gln
275 280 285

Val Arg Gly Lys Glu Glu Phe Asp Ile Gly Gln Trp Ser Lys Trp Ser
290 295 300

Pro Glu Val Thr Gly Thr Pro Trp Leu Ala Glu Pro Arg Thr Thr Pro
305 310 315 320

Ala Gly Ile Pro Gly Asn Pro Thr Gln Val Ser Val Glu Asp Tyr Asp
325 330 335

Asn His Glu Asp Gln Tyr Gly Ser Ser Thr Glu Ala Thr Ser Val Leu
340 345 350

Ala Pro Val Gln Gly Ser Ser Pro Ile Pro Leu Pro
355 360

<210> 11
<211> 1407
<212> DNA
<213> Human

<400> 11

atgctggccg tcggctgcgc gctgctggct gccctgctgg ccgcgccggg agcggcgctg 60
gccccaaggc gctgccctgc gcaggaggtg gcgagaggcg tgctgaccag tctgccagga 120
gacagcgtga ctctgacctg cccgggggta gagccggaag acaatgccac tgttcactgg 180
gtgctcagga agccggctgc aggctcccac cccagcagat gggctggcat gggaaggagg 240
ctgctgctga ggtcggtgca gctccacgac tctggaaact attcatgcta ccgggccggc 300
cgcccagctg ggactgtgca cttgctggtg gatgttcccc ccgaggagcc ccagctctcc 360
tgcttccgga agagcccct cagcaatgtt gtttgtgagt ggggtcctcg gagcacccca 420
tccctgacga caaaggctgt gctcttggtg aggaagtttc agaacagtcc ggccgaagac 480
ttccaggagc cgtgccagta ttcccaggag tcccagaagt tctcctgcca gttagcagtc 540
ccggagggag acagctcttt ctacatagtg tccatgtgcg tcgccagtag tgtcgggagc 600
aagttcagca aaactcaaac ctttcagggt tgtggaatct tgcagcctga tccgcctgcc 660
aacatcacag tcactgccgt ggccagaaac ccccgctggc tcagtgtcac ctggcaagac 720
ccccactcct ggaactcatc tttctacaga ctacggtttg agctcagata tcgggctgaa 780

cggtcaaaga cattcacaac atggatggtc aaggacctcc agcatcactg tgtcatccac 840
gacgcctgga gcggcctgag gcacgtggtg cagcttcgtg cccaggagga gttcgggcaa 900
ggcgagtgga gcgagtggag cccggaggcc atgggcacgc cttggacaga atccaggagt 960
cctccagctg agaacgaggt gtccaccccc atgcaggcac ttactactaa taaagacgat 1020
gataatattc tcttcagaga ttctgcaaat gcgacaagcc tcccagtgca agattcttct 1080
tcagtaccac tgcccacatt cctggttgct ggagggagcc tggccttcgg aacgctcctc 1140
tgcattgcca ttgttctgag gttcaagaag acgtggaagc tgcgggctct gaaggaaggc 1200
aagacaagca tgcatccgcc gtactctttg ggcagctgg tcccggagag gcctcgaccc 1260
accccagtgc ttgttcctct catctcccca ccggtgtccc ccagcagcct ggggtctgac 1320
aatacctcga gccacaaccg accagatgcc agggacccac ggagccctta tgacatcagc 1380
aatacagact acttcttccc cagatag 1407

<210> 12
<211> 365
<212> PRT
<213> Human

<400> 12

Met Leu Ala Val Gly Cys Ala Leu Leu Ala Ala Leu Leu Ala Ala Pro
1               5                   10                  15

Gly Ala Ala Leu Ala Pro Arg Arg Cys Pro Ala Gln Glu Val Ala Arg
            20                  25                  30

Gly Val Leu Thr Ser Leu Pro Gly Asp Ser Val Thr Leu Thr Cys Pro
        35                  40                  45

Gly Val Glu Pro Glu Asp Asn Ala Thr Val His Trp Val Leu Arg Lys
    50                  55                  60

Pro Ala Ala Gly Ser His Pro Ser Arg Trp Ala Gly Met Gly Arg Arg
65                  70                  75                  80

Leu Leu Leu Arg Ser Val Gln Leu His Asp Ser Gly Asn Tyr Ser Cys
                85                  90                  95

Tyr Arg Ala Gly Arg Pro Ala Gly Thr Val His Leu Leu Val Asp Val
            100                 105                 110

Pro Pro Glu Glu Pro Gln Leu Ser Cys Phe Arg Lys Ser Pro Leu Ser
        115                 120                 125

Asn Val Val Cys Glu Trp Gly Pro Arg Ser Thr Pro Ser Leu Thr Thr
130 135 140

Lys Ala Val Leu Leu Val Arg Lys Phe Gln Asn Ser Pro Ala Glu Asp
145 150 155 160

Phe Gln Glu Pro Cys Gln Tyr Ser Gln Glu Ser Gln Lys Phe Ser Cys
165 170 175

Gln Leu Ala Val Pro Glu Gly Asp Ser Ser Phe Tyr Ile Val Ser Met
180 185 190

Cys Val Ala Ser Ser Val Gly Ser Lys Phe Ser Lys Thr Gln Thr Phe
195 200 205

Gln Gly Cys Gly Ile Leu Gln Pro Asp Pro Pro Ala Asn Ile Thr Val
210 215 220

Thr Ala Val Ala Arg Asn Pro Arg Trp Leu Ser Val Thr Trp Gln Asp
225 230 235 240

Pro His Ser Trp Asn Ser Ser Phe Tyr Arg Leu Arg Phe Glu Leu Arg
245 250 255

Tyr Arg Ala Glu Arg Ser Lys Thr Phe Thr Thr Trp Met Val Lys Asp
260 265 270

Leu Gln His His Cys Val Ile His Asp Ala Trp Ser Gly Leu Arg His
275 280 285

Val Val Gln Leu Arg Ala Gln Glu Glu Phe Gly Gln Gly Glu Trp Ser
290 295 300

Glu Trp Ser Pro Glu Ala Met Gly Thr Pro Trp Thr Glu Ser Arg Ser
305 310 315 320

Pro Pro Ala Glu Asn Glu Val Ser Thr Pro Met Gln Ala Leu Thr Thr
325 330 335

Asn Lys Asp Asp Asp Asn Ile Leu Phe Arg Asp Ser Ala Asn Ala Thr
340 345 350

Ser Leu Pro Val Gln Asp Ser Ser Ser Val Pro Leu Pro
355 360 365

SEQUENCE LISTING

<110> Warner-Lambert Company LLC
Bove, Susan R

Kilgore, Kenneth

<120> Methods of Treating Osteoarthritis with IL-6 Antagonists

<130> PC32145A

<150> 60/543,814
<151> 2004-02-11

<150> PCT/IB2005/000240
<151> 2005-01-31

<160> 12

<170> PatentIn version 3.3

<210> 1
<211> 636
<212> DNA
<213> Mus musculus

<400> 1

```
atgaagttcc tctctgcaag agacttccat ccagttgcct tcttgggact gatgctggtg      60
acaaccacgg ccttccctac ttcacaagtc cggagaggag acttcacaga ggataccact     120
cccaacagac ctgtctatac cacttcacaa gtcggaggct taattacaca tgttctctgg     180
gaaatcgtgg aaatgagaaa agagttgtgc aatggcaatt ctgattgtat gaacaacgat     240
gatgcacttg cagaaaacaa tctgaaactt ccagagatac aaagaaatga tggatgctac     300
caaactggat ataatcagga aatttgccta ttgaaaattt cctctggtct tctggagtac     360
catagctacc tggagtacat gaagaacaac ttaaaagata acaagaaaga caaagccaga     420
gtccttcaga gagatacaga aactctaatt catatcttca accaagaggt aaaagattta     480
cataaaatag tccttcctac cccaatttcc aatgctctcc taacagataa gctggagtca     540
cagaaggagt ggctaaggac caagaccatc caattcatct tgaaatcact tgaagaattt     600
ctaaaagtca ctttgagatc tactcggcaa acctag                               636
```

<210> 2
<211> 211
<212> PRT
<213> Mus musculus

<400> 2

```
Met Lys Phe Leu Ser Ala Arg Asp Phe His Pro Val Ala Phe Leu Gly
1               5                   10                  15

Leu Met Leu Val Thr Thr Thr Ala Phe Pro Thr Ser Gln Val Arg Arg
            20                  25                  30

Gly Asp Phe Thr Glu Asp Thr Thr Pro Asn Arg Pro Val Tyr Thr Thr
        35                  40                  45

Ser Gln Val Gly Gly Leu Ile Thr His Val Leu Trp Glu Ile Val Glu
    50                  55                  60

Met Arg Lys Glu Leu Cys Asn Gly Asn Ser Asp Cys Met Asn Asn Asp
65                  70                  75                  80

Asp Ala Leu Ala Glu Asn Asn Leu Lys Leu Pro Glu Ile Gln Arg Asn
                85                  90                  95

Asp Gly Cys Tyr Gln Thr Gly Tyr Asn Gln Glu Ile Cys Leu Leu Lys
            100                 105                 110

Ile Ser Ser Gly Leu Leu Glu Tyr His Ser Tyr Leu Glu Tyr Met Lys
        115                 120                 125

Asn Asn Leu Lys Asp Asn Lys Lys Asp Lys Ala Arg Val Leu Gln Arg
    130                 135                 140

Asp Thr Glu Thr Leu Ile His Ile Phe Asn Gln Glu Val Lys Asp Leu
145                 150                 155                 160

His Lys Ile Val Leu Pro Thr Pro Ile Ser Asn Ala Leu Leu Thr Asp
                165                 170                 175

Lys Leu Glu Ser Gln Lys Glu Trp Leu Arg Thr Lys Thr Ile Gln Phe
            180                 185                 190

Ile Leu Lys Ser Leu Glu Glu Phe Leu Lys Val Thr Leu Arg Ser Thr
        195                 200                 205

Arg Gln Thr
    210
```

<210> 3
<211> 636
<212> DNA
<213> Rattus norvegicus

<400> 3

```
atgaagtttc tctccgcaag agacttccag ccagttgcct tcttgggact gatgttgttg    60
acagccactg ccttccctac ttcacaagtc cggagaggag acttcacaga ggataccacc   120
cacaacagac cagtatatac cacttcacaa gtcggaggct taattacata tgttctcagg   180
gagatcttgg aaatgagaaa agagttgtgc aatggcaatt ctgattgtat gaacagcgat   240
gatgcactgt cagaaaacaa tctgaaactt ccagaaatac aaagaaatga tggatgcttc   300
caaactggat ataaccagga aatttgccta ttgaaaatct gctctggtct tctggagttc   360
cgtttctacc tggagtttgt gaagaacaac ttacaagata acaagaaaga caaagccaga   420
gtcattcaga gcaatactga aaccctagtt catatcttca aacaagagat aaaagactca   480
tataaaatag tccttcctac cccaacttcc aatgctctcc taatggagaa gttagagtca   540
cagaaggagt ggctaaggac caagaccatc caactcatct tgaaagcact tgaagaattt   600
ctaaaggtca ctatgaggtc tactcggcaa acctag                             636
```

<210> 4
<211> 211
<212> PRT
<213> Rattus norvegicus

<400> 4

```
Met Lys Phe Leu Ser Ala Arg Asp Phe Gln Pro Val Ala Phe Leu Gly
1               5                   10                  15
```

```
Leu Met Leu Leu Thr Ala Thr Ala Phe Pro Thr Ser Gln Val Arg Arg
            20                  25                  30

Gly Asp Phe Thr Glu Asp Thr Thr His Asn Arg Pro Val Tyr Thr Thr
        35                  40                  45

Ser Gln Val Gly Gly Leu Ile Thr Tyr Val Leu Arg Glu Ile Leu Glu
    50                  55                  60

Met Arg Lys Glu Leu Cys Asn Gly Asn Ser Asp Cys Met Asn Ser Asp
65                  70                  75                  80

Asp Ala Leu Ser Glu Asn Asn Leu Lys Leu Pro Glu Ile Gln Arg Asn
                85                  90                  95

Asp Gly Cys Phe Gln Thr Gly Tyr Asn Gln Glu Ile Cys Leu Leu Lys
            100                 105                 110

Ile Cys Ser Gly Leu Leu Glu Phe Arg Phe Tyr Leu Glu Phe Val Lys
        115                 120                 125

Asn Asn Leu Gln Asp Asn Lys Lys Asp Lys Ala Arg Val Ile Gln Ser
    130                 135                 140

Asn Thr Glu Thr Leu Val His Ile Phe Lys Gln Glu Ile Lys Asp Ser
145                 150                 155                 160

Tyr Lys Ile Val Leu Pro Thr Pro Thr Ser Asn Ala Leu Leu Met Glu
                165                 170                 175

Lys Leu Glu Ser Gln Lys Glu Trp Leu Arg Thr Lys Thr Ile Gln Leu
            180                 185                 190

Ile Leu Lys Ala Leu Glu Glu Phe Leu Lys Val Thr Met Arg Ser Thr
        195                 200                 205

Arg Gln Thr
    210
```

<210> 5
<211> 639
<212> DNA
<213> Human

<400> 5

```
atgaactcct tctccacaag cgccttcggt ccagttgcct tctccctggg gctgctcctg      60
gtgttgcctg ctgccttccc tgccccagta cccccaggag aagattccaa agatgtagcc     120
gccccacaca gacagccact cacctcttca gaacgaattg acaaacaaat tcggtacatc     180
ctcgacggca tctcagccct gagaaaggag acatgtaaca agagtaacat gtgtgaaagc     240
agcaaagagg cactggcaga aaacaacctg aaccttccaa agatggctga aaaagatgga     300
tgcttccaat ctggattcaa tgaggagact tgcctggtga aaatcatcac tggtcttttg     360
gagtttgagg tatacctaga gtacctccag aacagatttg agagtagtga ggaacaagcc     420
agagctgtgc agatgagtac aaaagtcctg atccagttcc tgcagaaaaa ggcaaagaat     480
ctagatgcaa taaccacccc tgacccaacc acaaatgcca gcctgctgac gaagctgcag     540

gcacagaacc agtggctgca ggacatgaca actcatctca ttctgcgcag ctttaaggag     600
ttcctgcagt ccagcctgag ggctcttcgg caaatgtag                            639
```

<210> 6
<211> 212
<212> PRT
<213> Human

<400> 6

```
Met Asn Ser Phe Ser Thr Ser Ala Phe Gly Pro Val Ala Phe Ser Leu
1               5                   10                  15

Gly Leu Leu Leu Val Leu Pro Ala Ala Phe Pro Ala Pro Val Pro Pro
            20                  25                  30

Gly Glu Asp Ser Lys Asp Val Ala Ala Pro His Arg Gln Pro Leu Thr
        35                  40                  45

Ser Ser Glu Arg Ile Asp Lys Gln Ile Arg Tyr Ile Leu Asp Gly Ile
    50                  55                  60

Ser Ala Leu Arg Lys Glu Thr Cys Asn Lys Ser Asn Met Cys Glu Ser
65                  70                  75                  80

Ser Lys Glu Ala Leu Ala Glu Asn Asn Leu Asn Leu Pro Lys Met Ala
                85                  90                  95

Glu Lys Asp Gly Cys Phe Gln Ser Gly Phe Asn Glu Glu Thr Cys Leu
            100                 105                 110

Val Lys Ile Ile Thr Gly Leu Leu Glu Phe Glu Val Tyr Leu Glu Tyr
        115                 120                 125

Leu Gln Asn Arg Phe Glu Ser Ser Glu Glu Gln Ala Arg Ala Val Gln
    130                 135                 140

Met Ser Thr Lys Val Leu Ile Gln Phe Leu Gln Lys Lys Ala Lys Asn
145                 150                 155                 160

Leu Asp Ala Ile Thr Thr Pro Asp Pro Thr Thr Asn Ala Ser Leu Leu
                165                 170                 175

Thr Lys Leu Gln Ala Gln Asn Gln Trp Leu Gln Asp Met Thr Thr His
            180                 185                 190

Leu Ile Leu Arg Ser Phe Lys Glu Phe Leu Gln Ser Ser Leu Arg Ala
        195                 200                 205

Leu Arg Gln Met
    210
```

<210> 7
<211> 1323
<212> DNA
<213> Mus musculus

<400> 7

atgctgaccg tcggctgcac gctgttggtc gccctgctgg ccgcgcccgc ggtcgcgctg 60

gtcctcggga gctgccgcgc gctggaggtg gcaaatggca cagtgacaag cctgccaggg 120
gccaccgtta ccctgatttg ccccgggaag gaagcagcag gcaatgttac cattcactgg 180
gtgtactctg gctcacaaaa cagagaatgg actaccacag gaaacacact ggttctgagg 240
gacgtgcagc tcagcgacac tggggactat ttatgctccc tgaatgatca cctggtgggg 300
actgtgccct tgctggtgga tgttccccca gaggagccca agctctcctg cttccggaag 360
aacccccttg tcaacgccat ctgtgagtgg cgtccgagca gcacccccctc tccaaccacg 420
aaggctgtgc tgtttgcaaa gaaaatcaac accaccaacg ggaagagtga cttccaggtg 480
ccctgccagt attctcagca gctgaaaagc ttctcctgcc aggtggagat cctggagggt 540
gacaaagtat accacatagt gtcactgtgc gttgcaaaca gtgtgggaag caagtccagc 600
cacaacgaag cgtttcacag cttaaaaatg gtgcagccgg atccacctgc caaccttgtg 660
gtatcagcca tacctggaag gccgcgctgg ctcaaagtca gctggcagca ccctgagacc 720
tgggacccga gttactactt gctgcagttc cagcttcgat accgacctgt atggtcaaag 780
gagttcacgg tgttgctgct cccggtggcc cagtaccaat gcgtcatcca tgatgccttg 840
cgaggagtga agcacgtggt ccaggtccgt gggaaggagg agcttgacct tggccagtgg 900
agtgaatggt ccccagaggt cacgggcact ccttggatag cagagcccag gaccaccccg 960
gcaggaatcc tctggaaccc cacacaggtc tctgttgaag actctgccaa ccacgaggat 1020
cagtacgaaa gttctacaga agcaacgagt gtcctcgccc cagtgcaaga atcctcgtcc 1080
atgtccctgc ccacattcct ggtagctgga ggaagcttgg cgtttgggtt gcttctctgt 1140
gtcttcatca tcctgtgttg ggagccgcgc ccacattcgc cgttacaaga tggcgctgac 1200
agctgtgttc taagtggtaa acaaataatc tgcgcatgtg ccgagggtgg ttctccactc 1260
catgtgctct gccttccccg tgacgtcaac tcggccgatg ggctgcagcc aatcagggag 1320
tga 1323

<210> 8
<211> 364
<212> PRT
<213> Mus musculus

<400> 8

```
Met Leu Thr Val Gly Cys Thr Leu Leu Val Ala Leu Leu Ala Ala Pro
1               5                   10                  15

Ala Val Ala Leu Val Leu Gly Ser Cys Arg Ala Leu Glu Val Ala Asn
            20                  25                  30

Gly Thr Val Thr Ser Leu Pro Gly Ala Thr Val Thr Leu Ile Cys Pro
        35                  40                  45

Gly Lys Glu Ala Ala Gly Asn Val Thr Ile His Trp Val Tyr Ser Gly
    50                  55                  60

Ser Gln Asn Arg Glu Trp Thr Thr Thr Gly Asn Thr Leu Val Leu Arg
65                  70                  75                  80

Asp Val Gln Leu Ser Asp Thr Gly Asp Tyr Leu Cys Ser Leu Asn Asp
                85                  90                  95
```

```
His Leu Val Gly Thr Val Pro Leu Leu Val Asp Val Pro Pro Glu Glu
            100                 105                 110

Pro Lys Leu Ser Cys Phe Arg Lys Asn Pro Leu Val Asn Ala Ile Cys
        115                 120                 125

Glu Trp Arg Pro Ser Ser Thr Pro Ser Pro Thr Thr Lys Ala Val Leu
    130                 135                 140

Phe Ala Lys Lys Ile Asn Thr Thr Asn Gly Lys Ser Asp Phe Gln Val
145                 150                 155                 160

Pro Cys Gln Tyr Ser Gln Gln Leu Lys Ser Phe Ser Cys Gln Val Glu
                165                 170                 175

Ile Leu Glu Gly Asp Lys Val Tyr His Ile Val Ser Leu Cys Val Ala
            180                 185                 190

Asn Ser Val Gly Ser Lys Ser Ser His Asn Glu Ala Phe His Ser Leu
        195                 200                 205

Lys Met Val Gln Pro Asp Pro Pro Ala Asn Leu Val Val Ser Ala Ile
    210                 215                 220

Pro Gly Arg Pro Arg Trp Leu Lys Val Ser Trp Gln His Pro Glu Thr
225                 230                 235                 240

Trp Asp Pro Ser Tyr Tyr Leu Leu Gln Phe Gln Leu Arg Tyr Arg Pro
                245                 250                 255

Val Trp Ser Lys Glu Phe Thr Val Leu Leu Leu Pro Val Ala Gln Tyr
            260                 265                 270

Gln Cys Val Ile His Asp Ala Leu Arg Gly Val Lys His Val Val Gln
        275                 280                 285

Val Arg Gly Lys Glu Glu Leu Asp Leu Gly Gln Trp Ser Glu Trp Ser
    290                 295                 300

Pro Glu Val Thr Gly Thr Pro Trp Ile Ala Glu Pro Arg Thr Thr Pro
305                 310                 315                 320

Ala Gly Ile Leu Trp Asn Pro Thr Gln Val Ser Val Glu Asp Ser Ala
                325                 330                 335

Asn His Glu Asp Gln Tyr Glu Ser Ser Thr Glu Ala Thr Ser Val Leu
            340                 345                 350

Ala Pro Val Gln Glu Ser Ser Ser Met Ser Leu Pro
        355                 360
```

<210> 9
<211> 1389
<212> DNA
<213> Rattus norvegicus

<400> 9

```
atgctggccg tcggctgcac cctgctggtc gccctgctgg ccgcgcccgc agtcgcgctg      60
gtccttggga gctgccgcgc gctggaggtg gcaaatggta cggtgacgag cctgccaggg     120
gccactgtta ccctgatctg ccctgggaag gaagcagcag gcaatgctac cattcactgg     180
gtgtactcag gctcacagag cagagaatgg actaccacgg gaaacacact ggttctgagg     240
gccgtgcagg tcaatgacac tgggcactat ttgtgcttcc tggatgatca tctggttggg     300
actgtgccct tgctggtgga tgttcccca gaggagccca agctctcctg cttccggaag     360
aacccccttg taaatgcctt ttgtgagtgg catccaagca gcactccctc tccaaccacg     420
aaggctgtga tgtttgcaaa gaaaatcaac accaccaatg ggaagagtga cttccaggtg     480
ccttgccagt attctcagca gctgaaaagc ttctcctgcg aggtggagat cctggagggt     540
gacaaagtgt accacatagt gtcactgtgc gttgcaaaca gtgtcggaag caggtccagc     600
cacaatgtag tatttcagag tttaaaaatg gtgcagccgg atccacctgc caaccttgtg     660
gtatcagcca tacctggaag cctcgttggc tcaaagtcag ttggcaagac cctgagtcct     720
gggacccaag ttactacttg ttgcaattcg agcttcgata ccgacctgta tggtcaaaga     780
acgttcacgg tgtggccgct ccaggtggcc cagcatcaat gtgtcatcca tgatgccttg     840
cgaggagtaa agcatgtggt gcaggtccga gggaaggagg agtttgacat tggccagtgg     900
agcaaatggt ccccggaggt cacaggcact ccttggctag cagagcccag gaccactccg     960
gcagggatcc cggggaaccc cacacaggtc tctgttgaag actatgacaa ccacgaggat    1020
cagtacggaa gttctacaga agcaacgagt gtcctcgccc cagtgcaagg atcctcgcct    1080
atacccctgc ccacattcct ggtagctgga ggaagcctgg cgtttggatt gcttctctgt    1140
gtcttcatca tcttgagact caagaagaaa tggaagtcac aggctgagaa ggaaagcaag    1200
acgacttctc ccccaccgta tcccttggga ccgctgaagc cgaccttcct cctggttcct    1260
ctcctcaccc catcagggtc ccataacagc tctgggactg acaacaccgg aagccacagc    1320
tgcctgggtg tcagggaccc acagtgccct aatgacaaca gcaacagaga ctacttattc    1380
cccagataa                                                           1389
```

<210> 10
<211> 364
<212> PRT
<213> Rattus norvegicus

<400> 10

```
Met Leu Ala Val Gly Cys Thr Leu Leu Val Ala Leu Leu Ala Ala Pro
1               5                   10                  15

Ala Val Ala Leu Val Leu Gly Ser Cys Arg Ala Leu Glu Val Ala Asn
            20                  25                  30

Gly Thr Val Thr Ser Leu Pro Gly Ala Thr Val Thr Leu Ile Cys Pro
        35                  40                  45

Gly Lys Glu Ala Ala Gly Asn Ala Thr Ile His Trp Val Tyr Ser Gly
    50                  55                  60

Ser Gln Ser Arg Glu Trp Thr Thr Thr Gly Asn Thr Leu Val Leu Arg
65                  70                  75                  80

Ala Val Gln Val Asn Asp Thr Gly His Tyr Leu Cys Phe Leu Asp Asp
                85                  90                  95
```

```
His Leu Val Gly Thr Val Pro Leu Leu Val Asp Val Pro Pro Glu Glu
            100                 105                 110

Pro Lys Leu Ser Cys Phe Arg Lys Asn Pro Leu Val Asn Ala Phe Cys
        115                 120                 125

Glu Trp His Pro Ser Ser Thr Pro Ser Pro Thr Thr Lys Ala Val Met
    130                 135                 140

Phe Ala Lys Lys Ile Asn Thr Thr Asn Gly Lys Ser Asp Phe Gln Val
145                 150                 155                 160

Pro Cys Gln Tyr Ser Gln Gln Leu Lys Ser Phe Ser Cys Glu Val Glu
                165                 170                 175

Ile Leu Glu Gly Asp Lys Val Tyr His Ile Val Ser Leu Cys Val Ala
            180                 185                 190

Asn Ser Val Gly Ser Arg Ser Ser His Asn Val Val Phe Gln Ser Leu
        195                 200                 205

Lys Met Val Gln Pro Asp Pro Pro Ala Asn Leu Val Val Ser Ala Ile
    210                 215                 220

Pro Gly Ser Leu Val Gly Ser Lys Ser Val Gly Lys Thr Leu Ser Pro
225                 230                 235                 240

Gly Thr Gln Val Thr Thr Cys Cys Asn Ser Ser Phe Asp Thr Asp Leu
                245                 250                 255

Tyr Gly Gln Arg Thr Phe Thr Val Trp Pro Leu Gln Val Ala Gln His
            260                 265                 270

Gln Cys Val Ile His Asp Ala Leu Arg Gly Val Lys His Val Val Gln
        275                 280                 285

Val Arg Gly Lys Glu Glu Phe Asp Ile Gly Gln Trp Ser Lys Trp Ser
    290                 295                 300

Pro Glu Val Thr Gly Thr Pro Trp Leu Ala Glu Pro Arg Thr Thr Pro
305                 310                 315                 320

Ala Gly Ile Pro Gly Asn Pro Thr Gln Val Ser Val Glu Asp Tyr Asp
                325                 330                 335

Asn His Glu Asp Gln Tyr Gly Ser Ser Thr Glu Ala Thr Ser Val Leu
            340                 345                 350

Ala Pro Val Gln Gly Ser Ser Pro Ile Pro Leu Pro
        355                 360
```

<210> 11

<211> 1407
<212> DNA
<213> Human

<400> 11

```
atgctggccg tcggctgcgc gctgctggct gccctgctgg ccgcgccggg agcggcgctg      60
gccccaaggc gctgccctgc gcaggaggtg gcgagaggcg tgctgaccag tctgccagga     120
gacagcgtga ctctgacctg cccgggggta gagccggaag acaatgccac tgttcactgg     180
gtgctcagga agccggctgc aggctcccac cccagcagat gggctggcat gggaaggagg     240
ctgctgctga ggtcggtgca gctccacgac tctggaaact attcatgcta ccgggccggc     300
cgcccagctg ggactgtgca cttgctggtg gatgttcccc ccgaggagcc ccagctctcc     360
tgcttccgga agagccccct cagcaatgtt gtttgtgagt ggggtcctcg gagcacccca     420
tccctgacga caaaggctgt gctcttggtg aggaagtttc agaacagtcc ggccgaagac     480
ttccaggagc cgtgccagta ttcccaggag tcccagaagt tctcctgcca gttagcagtc     540
ccggagggag acagctcttt ctacatagtg tccatgtgcg tcgccagtag tgtcgggagc     600
aagttcagca aaactcaaac ctttcagggt tgtggaatct tgcagcctga tccgcctgcc     660
aacatcacag tcactgccgt ggccagaaac ccccgctggc tcagtgtcac ctggcaagac     720
ccccactcct ggaactcatc tttctacaga ctacggtttg agctcagata tcgggctgaa     780
cggtcaaaga cattcacaac atggatggtc aaggacctcc agcatcactg tgtcatccac     840
gacgcctgga gcggcctgag gcacgtggtg cagcttcgtg cccaggagga gttcgggcaa     900
ggcgagtgga gcgagtggag cccggaggcc atgggcacgc cttggacaga atccaggagt     960
cctccagctg agaacgaggt gtccaccccc atgcaggcac ttactactaa taaagacgat    1020
gataatattc tcttcagaga ttctgcaaat gcgacaagcc tcccagtgca agattcttct    1080
tcagtaccac tgcccacatt cctggttgct ggagggagcc tggccttcgg aacgctcctc    1140
tgcattgcca ttgttctgag gttcaagaag acgtggaagc tgcgggctct gaaggaaggc    1200
aagacaagca tgcatccgcc gtactctttg ggcagctgg tcccggagag gcctcgaccc     1260
accccagtgc ttgttcctct catctcccca ccggtgtccc ccagcagcct ggggtctgac    1320
aatacctcga gccacaaccg accagatgcc agggacccac ggagccctta tgacatcagc    1380
aatacagact acttcttccc cagatag                                        1407
```

<210> 12
<211> 365
<212> PRT
<213> Human

<400> 12

Met Leu Ala Val Gly Cys Ala Leu Leu Ala Ala Leu Leu Ala Ala Pro
1 5 10 15

Gly Ala Ala Leu Ala Pro Arg Arg Cys Pro Ala Gln Glu Val Ala Arg
20 25 30

Gly Val Leu Thr Ser Leu Pro Gly Asp Ser Val Thr Leu Thr Cys Pro
35 40 45

Gly Val Glu Pro Glu Asp Asn Ala Thr Val His Trp Val Leu Arg Lys
50 55 60

Pro Ala Ala Gly Ser His Pro Ser Arg Trp Ala Gly Met Gly Arg Arg
65 70 75 80

```
Leu Leu Leu Arg Ser Val Gln Leu His Asp Ser Gly Asn Tyr Ser Cys
                85                  90                  95

Tyr Arg Ala Gly Arg Pro Ala Gly Thr Val His Leu Leu Val Asp Val
            100                 105                 110

Pro Pro Glu Glu Pro Gln Leu Ser Cys Phe Arg Lys Ser Pro Leu Ser
        115                 120                 125

Asn Val Val Cys Glu Trp Gly Pro Arg Ser Thr Pro Ser Leu Thr Thr
    130                 135                 140

Lys Ala Val Leu Leu Val Arg Lys Phe Gln Asn Ser Pro Ala Glu Asp
145                 150                 155                 160

Phe Gln Glu Pro Cys Gln Tyr Ser Gln Glu Ser Gln Lys Phe Ser Cys
                165                 170                 175

Gln Leu Ala Val Pro Glu Gly Asp Ser Ser Phe Tyr Ile Val Ser Met
            180                 185                 190

Cys Val Ala Ser Ser Val Gly Ser Lys Phe Ser Lys Thr Gln Thr Phe
        195                 200                 205

Gln Gly Cys Gly Ile Leu Gln Pro Asp Pro Pro Ala Asn Ile Thr Val
    210                 215                 220

Thr Ala Val Ala Arg Asn Pro Arg Trp Leu Ser Val Thr Trp Gln Asp
225                 230                 235                 240

Pro His Ser Trp Asn Ser Ser Phe Tyr Arg Leu Arg Phe Glu Leu Arg
                245                 250                 255

Tyr Arg Ala Glu Arg Ser Lys Thr Phe Thr Thr Trp Met Val Lys Asp
            260                 265                 270

Leu Gln His His Cys Val Ile His Asp Ala Trp Ser Gly Leu Arg His
        275                 280                 285

Val Val Gln Leu Arg Ala Gln Glu Glu Phe Gly Gln Gly Glu Trp Ser
    290                 295                 300

Glu Trp Ser Pro Glu Ala Met Gly Thr Pro Trp Thr Glu Ser Arg Ser
305                 310                 315                 320

Pro Pro Ala Glu Asn Glu Val Ser Thr Pro Met Gln Ala Leu Thr Thr
                325                 330                 335

Asn Lys Asp Asp Asp Asn Ile Leu Phe Arg Asp Ser Ala Asn Ala Thr
            340                 345                 350

Ser Leu Pro Val Gln Asp Ser Ser Ser Val Pro Leu Pro
        355                 360                 365
```